(19) **Europäisches Patentamt / European Patent Office / Office européen des brevets**

(11) **EP 3 441 749 B1**

(12) # EUROPEAN PATENT SPECIFICATION

(45) Date of publication and mention of the grant of the patent:
**22.11.2023 Bulletin 2023/47**

(51) International Patent Classification (IPC):
**G01N 21/3586** (2014.01)    **G01N 21/17** (2006.01)
**G01N 21/21** (2006.01)    **A61F 2/08** (2006.01)
**H01L 31/09** (2006.01)    **G01N 33/483** (2006.01)
**G01N 21/19** (2006.01)

(21) Application number: **17779151.4**

(22) Date of filing: **04.04.2017**

(52) Cooperative Patent Classification (CPC):
**G01N 21/3586; A61F 2/08; G01N 21/1717; G01N 21/21; G01N 33/4833;** G01N 21/19

(86) International application number:
**PCT/JP2017/014149**

(87) International publication number:
**WO 2017/175773 (12.10.2017 Gazette 2017/41)**

(54) **METHOD FOR OBSERVING DYNAMIC PHYSICAL PROPERTY OF BIOLOGICAL TISSUE, AND DEVICE FOR OBSERVING DYNAMIC PHYSICAL PROPERTY OF BIOLOGICAL TISSUE**

VERFAHREN ZUR BEOBACHTUNG EINER DYNAMISCH-PHYSIKALISCHEN EIGENSCHAFT VON BIOLOGISCHEM GEWEBE UND VORRICHTUNG ZUR BEOBACHTUNG EINER DYNAMISCH-PHYSIKALISCHEN EIGENSCHAFT VON BIOLOGISCHEM GEWEBE

PROCÉDÉ D'OBSERVATION DE PROPRIÉTÉ PHYSIQUE DYNAMIQUE DE TISSU BIOLOGIQUE, ET DISPOSITIF D'OBSERVATION DE PROPRIÉTÉ PHYSIQUE DYNAMIQUE DE TISSU BIOLOGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.04.2016 JP 2016075572**

(43) Date of publication of application:
**13.02.2019 Bulletin 2019/07**

(73) Proprietors:
• **Advanced Bio-Spectroscopy Co., Ltd.**
  **Tokyo 196-0033 (JP)**
• **The University Of Tokyo**
  **Tokyo 113-8654 (JP)**

(72) Inventors:
• **USHIDA, Takashi**
  **Tsukuba-shi**
  **Ibaraki 305-0817 (JP)**
• **FURUKAWA, Katsuko**
  **Tokyo 114-0015 (JP)**

• **NISHIZAWA, Seizi**
  **Akishima-shi**
  **Tokyo 196-0033 (JP)**

(74) Representative: **Lippert Stachow Patentanwälte Rechtsanwälte Partnerschaft mbB**
  **Frankenforster Strasse 135-137**
  **51427 Bergisch Gladbach (DE)**

(56) References cited:
**EP-A1- 1 801 939**    **JP-A- 2006 098 294**
**JP-A- 2012 202 812**    **US-A1- 2007 222 988**
**US-A1- 2014 224 021**    **US-A1- 2015 008 326**

• **A. Y. ELEZZABI ET AL: "Optical activity in an artificial chiral media: a terahertz time-domain investigation of Karl F Lindman's 1920 pioneering experiment", OPTICS EXPRESS, vol. 17, no. 8, 13 April 2009 (2009-04-13), pages 6600-6612, XP055639073, DOI: 10.1364/OE.17.006600**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 441 749 B1

- **HANGYO MASANORI: "Development and versatile applications of terahertz time-domain spectroscopy", 2014 39TH INTERNATIONAL CONFERENCE ON INFRARED, MILLIMETER, AND TERAHERTZ WAVES (IRMMW-THZ), IEEE, 14 September 2014 (2014-09-14), pages 1-4, XP032683607, DOI: 10.1109/IRMMW-THZ.2014.6955988 [retrieved on 2014-11-13]**
- **TAKASHI NOTAKE et al.: "Hacho Kahen Terahertz- ha Kogen o Mochiita Bunko Stokes Henko Keisoku System no Kaihatsu", Extended Abstracts, Japan Society of Applied Physics and Related Societies, 2012, pages 04-201, XP9514968,**
- **HIROAKI MINAMIDE et al.: "A Continuously Tunable Ring-Cavity THz-Wave Parametric Oscillator", The Review of Laser Engineering, vol. 29, no. 11, November 2001 (2001-11), pages 744-748, XP055429564,**
- **DORADLA PALLAVI ET AL: "Terahertz polarization imaging for colon cancer detection", PROCEEDINGS OF SPIE, IEEE, US, vol. 8985, 7 March 2014 (2014-03-07), pages 89850K-89850K, XP060034995, DOI: 10.1117/12.2038650 ISBN: 978-1-62841-730-2**
- **Kan Wai-Chi ET AL: "Terahertz pulsed imaging of knee cartilage", Biomedical optics express, vol. 1, no. 3, 20 September 2010 (2010-09-20), pages 967-974, XP055827962, United States ISSN: 2156-7085, DOI: 10.1364/BOE.1.000967 Retrieved from the Internet: URL:https://www.osapublishing.org/DirectPDFAccess/0B8EAC68-48D3-4BA1-936BE3A066C9811**
**6_205807/boe-1-3-967.pdf?da=1&id=205807&seq=0&mobile=no>**

## Description

[Technical Field]

The present invention relates to a method for observing a dynamic physical property of a biological tissue and a device for observing a dynamic physical property of a biological tissue.

[Background Art]

As society experiences aging of population, the number of patients suffering from diseases of organs of locomotion as represented by locomotive syndrome, in particular joint diseases has been increasing. One of the joint diseases is osteoarthritis. Osteoarthritis is rarely healed spontaneously once a patient develops it, which generally progresses irreversibly and makes it difficult for him/her to walk. Therefore, osteoarthritis is one of the major factors lowering the quality of life of middle-aged and elderly people.

From the viewpoint of, for example, maintenance of the patient's quality of life, there has been increasing needs for early diagnosis and early treatment of osteoarthritis. In recent years, as an early treatment of osteoarthritis, treatment by regenerated cartilage transplantation is performed.

[Citation List]

[Patent Literature]

[PTL 1] Japanese Unexamined Patent Application, Publication No. 2001-141567

[Summary of Invention]

[Technical Problem]

Regenerated tissue products such as regenerated cartilage involve a unique problem that they cannot be subjected to destruction inspection. As a result, in the current situations, regenerated tissues are transplanted without evaluation of their quality. Accordingly, development of a technique for noninvasively evaluating the regenerated tissues has been sought for.

Cartilage tissues are composed of chondrocytes and an extracellular matrix. In the extracellular matrix, macromolecules such as collagen, hyaluronic acid, sulfated glycosaminoglycan exist. The extracellular matrix constructs highly hydrated tissues by trapping a lot of water molecules and achieves high viscoelasticity of cartilage tissue and the like.

Regenerated cartilage uses chondrocytes collected and cultured from a living body and formed by a three-dimensional culture carrier, the chondrocytes, and the matrix produced by the chondrocytes.

Living tissues such as cartilage tissue and regenerated tissues such as regenerated cartilage are complex in structure and difficult to analyze. Patent Literature 1 discloses techniques for measuring an object to be measured such as a biopolymer which has been difficult to measure by conventional spectroscopic methods. However, in Patent Literature 1, it has not been possible to evaluate biological tissues such as cartilage tissue and regenerated tissues such as regenerated cartilage.

A. Y. Elezzabi ET AL: "Optical activity in an artificial chiral media: a terahertz time-domain investigation of Karl F Lindman's 1920 pioneering experiment", Optics Express, vol. 17, no. 8, 13 April 2009, pages 6600-6612, XP055639073. A polarization-sensitive THz time-domain spectroscopy step is employed for performing the experiments. The artificial chiral media being examined are composed of Au-coated Cu helices to study circular dichroism thereof to improve understanding of large chiral molecules.

HANGYO MASANORI: "Development and versatile applications of terahertz time-domain spectroscopy", 2014 39TH INTERNATIONAL CONFERENCE ON INFRARED, MILLIMETER, AND TERAHERTZ WAVES (IRMMW-THZ), IEEE, 14 September 2014, pages 1-4, XP032683607, gives an overview of progress of THz-TDS spectroscopy and expectations of use in various fields from basic science to security and industry. THz-TDS spectroscopy may be applied to the degradation diagnosis of ultra-high weight polyethylene, protein dynamics, dynamics of ionic liquids, inspection of explosives and inflammable liquids in plastic bottles.

US 2015/008326 A1 discloses an electromagnetic wave pulse measuring device, including an elastic vibration wave generating section which irradiates a predetermined area of a sample with an elastic vibration wave, an electromagnetic wave pulse generating section which irradiates the predetermined area, which is irradiated with the elastic vibration wave, with an electromagnetic wave pulse, and an electromagnetic wave pulse detecting section which measures a waveform of the electromagnetic wave pulse modulated in the predetermined area by the elastic vibration wave. An ultrasound wave is generated toward a measurement region of a sample.

US 2014/224021 A1 discloses an imaging system for an object such as human or animal tissue which applies acoustic vibrations localised in two or three dimensions and simultaneously illuminates the object with an illuminating electromagnetic wave. The acoustic vibration comprises a carrier wave that is amplitude modulated by an AM waveform.

EP 1 801 939 A1 discloses an infrared light radiation device, infrared light detection device, time-series conversion pulse spectrometer and infrared light radiation method. The infrared light emitting device is capable of polarizing emission light without causing loss of the emission light and having a simple configuration. A radiation means having a photoconductive layer receiving a pulse excitation light and generating a photocarrier, a pair of first antenna electrodes formed an the photoconductive film and facing each other via a gap, and a pair of second antenna electrodes arranged so as to face each other via the gap and arranged so as to have an angle relative to the first antenna electrode films are provided.

The present invention has been made in view of such circumstances, and it is an object of the present invention to provide a technique capable of evaluating biological tissues such as cartilage tissue and regenerated tissues such as regenerated cartilage for noninvasively evaluation of their quality, especially before transplantation.

[Solution to Problem]

The object is solved by a method according to claim 1 and by providing a device for application of said method according to claim 3. An advantageous embodiment of the method is claimed by caim 2. The present invention provides a method for observing a dynamic physical property of a biological tissue by irradiating the biological tissue with a pulsed light having a wavelength of a far-infrared wavelength region to observe the dynamic physical property of the biological tissue using vibrational optical activity spectroscopy, , the method comprising vibrating the biological tissue by vibrating a holding means (15) using a piezoelectric element, the holding means configured to hold the biological tissue when the biological tissue is irradiated with the pulsed light.

A cartilage tissue is a tissue containing a lot of water. Water molecules trapped in the extracellular matrix exist not as free water but as confined water in a state trapped and oriented in macromolecules such as the extracellular matrix.

The inventors of the present invention focused on the fact that the confinement of water molecules appears as a viscosity against the deformation due to compressive stress applied to the cartilage tissue, though that the degree of tissue formation and the dynamic physical property of the cartilage tissue could be estimated by measuring the state of the water molecules in the cartilage tissue, and conducted examinations and experimentations.

Vibration optical activity (abbreviated as VOA) spectroscopy is a scheme for measuring the difference in spectra for left and right circular polarized lights indicated by optically active molecules and examining steric information of molecules together with information on the vibration spectrum.

A terahertz wave resides in the far-infrared region and has good transmittivity to biological tissues. Since terahertz waves overlap with energy bands of intermolecular vibration and intramolecular vibration, it is possible to obtain information on tissue formation of biological tissues including cartilage tissue.

The terahertz wave has low photon energy and difficult to detect because it is hidden by noise in intensity or energy spectrum. As a result of intensive research, the inventors of the present invention have found that by using vibration optical activity spectroscopy in the terahertz band, it is made possible to obtain a spectrum attributable to the motion modes of water molecules (slow Debye relaxation mode, early Debye relaxation mode, intermolecular stretching vibration mode, and intermolecular declination vibration mode).

When the biological tissue is irradiated with a pulsed light, the biological tissue is vibrated. By forcibly imparting vibration to the biological tissue to cause the biological tissue to vibrate, it is made possible to perform spectroscopic measurement in accordance with the vibration frequency for imparting the vibration and gather an increased amount of information on the biological tissue at the molecule level. It is preferable to sweep the vibration frequency within a predetermined range.

For example, when a relaxation time of the biological tissue is to be obtained as the dynamic physical property, the vibration frequency dependence of the relaxation time can be revealed, which contributes to elucidation of the structure of the biological tissue.

In accordance with one aspect of the above-described invention, a radiation means is used, which has a photoconductive film receiving a pulse excitation light and generating a photocarrier and a pair of antenna electrode films formed on the photoconductive film and facing each other via a gap, wherein the radiation means is configured to emit the pulsed light having the wavelength of the far-infrared wavelength region by pulsed laser light irradiation; the biological tissue is irradiated with the pulsed light from the radiation means while vibrating the biological tissue; and the dynamic physical property of the biological tissue is observed based on a vibrational circular dichroism spectrum and/or polarization spectroscopy spectrum obtained from a time-series signal of a reflected pulsed light reflected by the biological tissue or a transmitted pulsed light transmitted through the biological tissue.

In addition, the present invention provides a device for observing a dynamic physical property of a biological tissue. The device includes a radiation means including a photoconductive film receiving an excitation pulsed laser light and generating a photocarrier and an antenna electrode film formed on the photoconductive film, and configured to emit a pulsed light having a wavelength of a far-infrared wavelength region by irradiation of the excitation pulsed laser light; a holding means configured to hold the biological tissue; a vibration device configured to vibrate the holding means; a detection means including a photoconductive film and an antenna electrode film and configured to obtain a time-series signal of a reflected pulsed light reflected by the biological tissue or a transmitted pulsed light transmitted through the biological tissue; and a control means configured to observe the dynamic physical property of the biological tissue based on a vibrational circular dichroism spectrum and/or polarization spectroscopy spectrum obtained from a signal obtained from the detection means.

Vibrations are forcibly imparted to the holding means holding the biological tissue with a vibration device. By virtue of this, it is made possible to perform spectroscopic measurement in accordance with the vibration frequency for imparting the vibrations and gather information on the biological tissue at the molecule level. It is preferable to sweep the vibration frequency within a predetermined range.

For example, when a relaxation time of the biological tissue is to be obtained as the dynamic physical property, the vibration frequency dependence of the relaxation time can be revealed, which contributes to the elucidation of the structure of the biological tissue.

As a device for imparting the vibrations, piezoelectric elements such as a PZT element are used.

Preferably, it is preferable that the holding means includes a metamaterial. By virtue of this, the measurement sensitivity can be increased. The metamaterial is a substance in which minute units called "unit elements" are artificially arranged with equal intervals at distances sufficiently smaller than the wavelength of electromagnetic waves and the metamaterial is configured to behave as a homogeneous medium for electromagnetic waves.

[Advantageous Effects of Invention]

According to the present invention, it is made possible to provide a technique for observing the degree of tissue formation and dynamic physical property of biological tissues such as cartilage tissue and regenerated tissues such as regenerated cartilage based on vibrational optical activity obtained by using terahertz waves.

In addition, since the biological tissue is vibrated while being irradiated with the pulsed light, it is made possible to perform spectroscopic measurement in accordance with the vibration frequency for imparting the vibration and gather an increased amount of information on the biological tissue at the molecule level.

[Brief Description of Drawings]

[Fig. 1] Fig. 1 is a schematic diagram of a device for observing a dynamic physical property of a biological tissue according to one embodiment of the present invention.
[Fig. 2] Fig. 2 is a plan view of a pattern of an antenna electrode film.
[Fig. 3] Fig. 3 is a diagram illustrating a polarized pulsed light.
[Fig. 4] Fig. 4 is a configuration diagram in which an observation unit optical system is illustrated.
[Fig. 5] Fig. 5 is a longitudinal cross-sectional view illustrating a holding means.

[Fig. 6] Fig. 6 is a graph illustrating an example of measured data.
[Fig. 7] Fig. 7 is a graph obtained by data processing using two-dimensional correlation spectroscopy.

[Description of Embodiments]

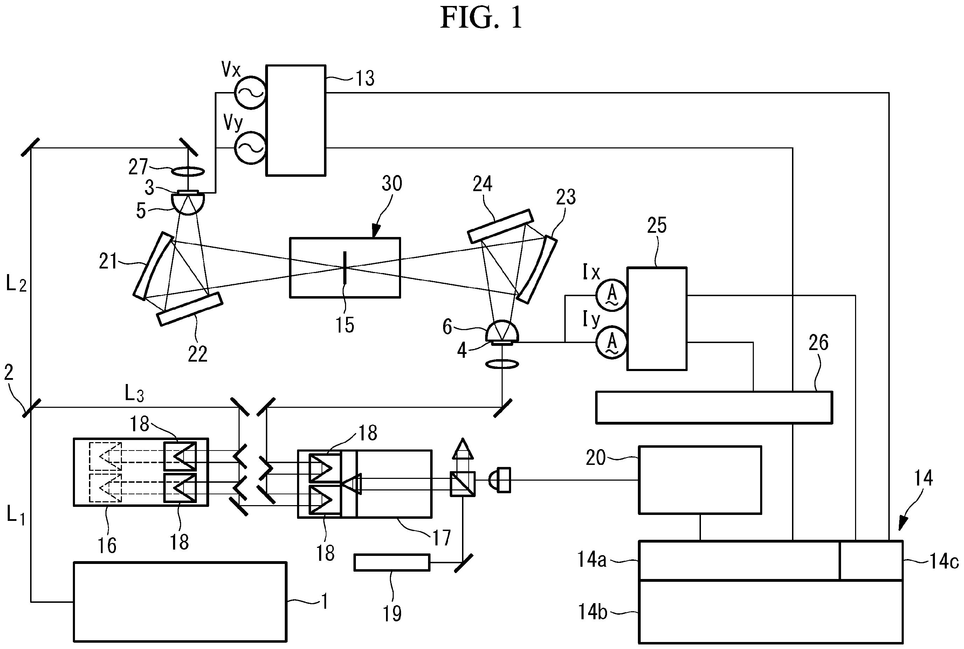

Fig. 1 illustrates an example of a device for observing a dynamic physical property of a biological tissue according to the present embodiment.

The device for observing the dynamic physical property of the biological tissue includes a pulsed laser light source 1. An ultrashort pulsed laser is used to configure the pulsed laser light source 1. The ultrashort pulsed laser may include a femtosecond fiber laser, a femtosecond mode-locked titanium sapphire laser, and the like.

The femtosecond fiber laser is configured, for example, by an LD-pumped passive mode-locked fiber laser using a 1.55 um band erbium (Er) doped fiber laser as a laser gain medium. The femtosecond fiber laser may be an ytterbium (Yb) doped fiber with relatively wide band and high quantum effect around 1.06 $\mu$m.

The femtosecond fiber laser is used, for example, at a center oscillation wavelength (second harmonic output) of 780 nm, a pulse width of 120 to 75 fs (femtoseconds), an average output of 30 mW, and a cyclic frequency of around 40 MHz.

In the femtosecond mode-locked titanium sapphire laser, a titanium doped sapphire (Ti:Al$_2$O$_3$) crystal is used as the laser medium. Titanium doped sapphire crystal is superior for stable oscillation of femtosecond pulses.

The femtosecond mode-locked titanium sapphire laser is used, for example, at a central oscillation wavelength of 780 nm, a pulse width of 100 to 45 fs, an average output of 100 mW, and a cyclic frequency of about 40 to 80 MHz.

In comparison with femtosecond mode-locked titanium sapphire laser, the femtosecond fiber laser has superior advantages in practical use in terms of its small size, light weight, simple stable operation, low cost, and low power consumption. On the other hand, the femtosecond mode-locked titanium-sapphire laser has advantages such as relatively wide spectral bandwidth compared with that of the femtosecond fiber laser, excellent ultrashort pulsed light oscillation, and facilitated high-output oscillation.

The device for observing the dynamic physical property of the biological tissue further includes a beam splitter (splitting means) 2 configured to split the femtosecond laser light (pulsed laser light) L$_1$ emitted from the pulsed laser light source 1 into pulsed laser light L$_2$ for use in excitation and pulsed laser light L$_3$ for use in detection.

The device for observing the dynamic physical property of the biological tissue further includes a terahertz wave generation element (radiation means) 3 configured to emit a pulsed light having a wavelength of a far-infrared wavelength region by irradiation of the pulsed laser light $L_2$ for excitation and a detection element (detection means) 4 configured to detect a time-series signal of the electric field intensity of a transmitted pulsed light (or reflected pulsed light) from the biological tissue irradiated with the pulsed light from the terahertz wave generation element 3. Super-hemispherical silicon lenses 5, 6 are arranged on the light emission side of the terahertz wave generation element 3 and the light incident side of the detection element 4, respectively, as illustrated in Fig. 1.

The terahertz wave generation element 3 and the detection element 4 are photoconductive antenna (abbreviated as PCA) elements. The PCA elements include a photoconductive film and an antenna electrode film.

The photoconductive film is formed, for example, by laminating a thin film of low temperature grown gallium arsenide (abbreviated as LT-GaAs) upon a semi-insulating gallium arsenide (abbreviated as SI-GaAs) substrate.

The antenna electrode film is laminated on the LT-GaAs photoconductive film. The material of the antenna electrode film may include gold (Au), etc. The antenna electrode film can be formed on the LT-GaAs thin layer by a vapor-deposition technique.

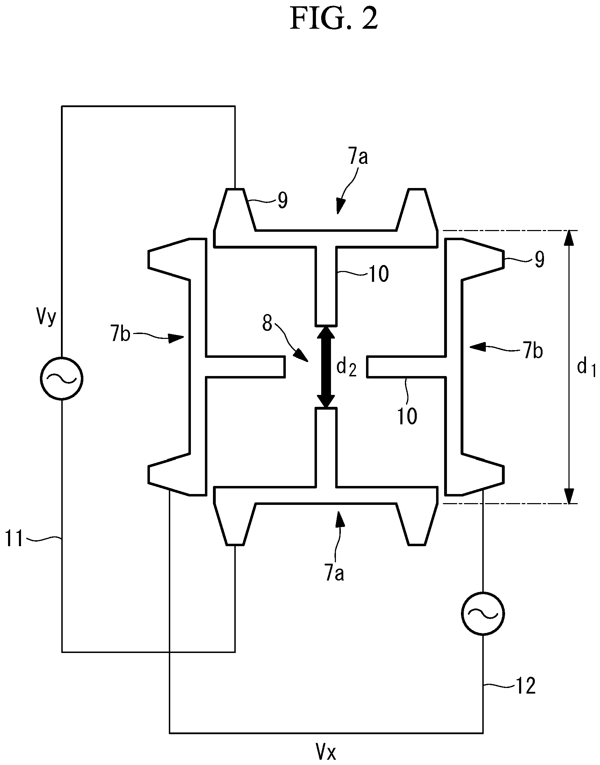

Fig. 2 is a plan view illustrating a pattern of the antenna electrode film according to the present embodiment. The antenna electrode film is configured by a pair of first antenna electrode films 7a arranged to face each other via a gap 8 and a pair of or multiple pairs of second antenna electrode films 7b arranged to face each other via the gap 8. The gap 8 is shared by the first antenna electrode films 7a and the second antenna electrode films 7b.

The pair of second antenna electrode films 7b is arranged with an angle with respect to the pair of first antenna electrode films 7a. In Fig. 2, the second antenna electrode films 7b in a plan view are arranged with an orientation different by substantially 90 degrees with respect to (substantially orthogonal to) the pair of first antenna electrode films 7a. Specifically, in the antenna electrode film, the pair of first antenna electrode films 7a and the pair of second antenna electrode films 7b have an orthogonal two-axis structure.

The first antenna electrode film 7a and the second antenna electrode film 7b are each configured by a conductive transmission channel 9 and a discharge electrode 10. The tip of the discharge electrode 10 is oriented toward the gap 8.

The pattern of the antenna electrode films can be adopted as appropriate depending on the frequency band of the terahertz wave to be emitted. For example, the outer-path interval $d_1$ of the conductive transmission channel 9 is defined as 30 μm and the gaps $d_2$ of the discharge electrodes 10 facing each other are both defined as 3 μm.

A first lead 11 is connected to the conductive transmission channel 9 of the first antenna electrode film 7a such that voltage can be applied to the pair of first antenna electrode films 7a. A second lead 12 is connected to the conductive transmission channel 9 of the second antenna electrode film 7b such that voltage can be applied to the pair of second antenna electrode films 7b. The other ends of the first lead and the second lead are connected to the modulation means 13 illustrated in Fig. 1.

The modulation means 13 has a voltage generation device (voltage generation unit) capable of applying bias voltage independently to both the first antenna electrode film 7a and the second antenna electrode film 7b. The modulation means 13 is capable of receiving signals from the control means 14 and modulating the phase, the amplitude, and the cyclic frequency.

A holding means 15 configured to hold a biological tissue (sample) is provided in an observation unit optical system 30 between the terahertz wave generation element 3 and the detection element 4. The term "biological tissue" may include biological tissues such as cartilage tissue and regenerated tissues such as regenerated cartilage.

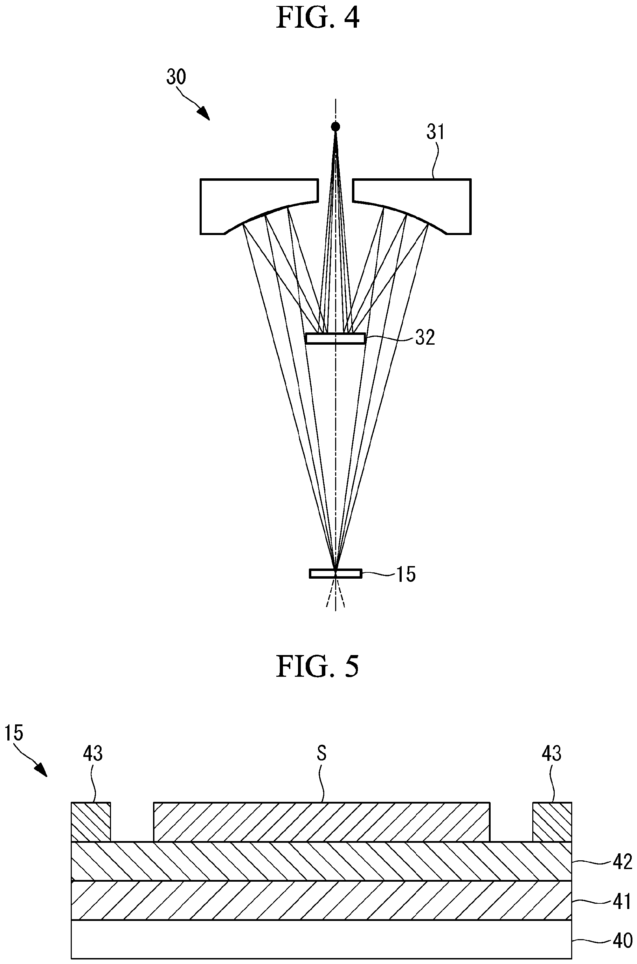

Fig. 4 illustrates an example of the observation unit optical system 30. It should be noted that, while the optical axis in Fig. 4 extends in the longitudinal direction, which differs from the example illustrated in Fig. 1, the example of Fig. 4 can also be adopted with its optical axis extending in the width direction as illustrated in Fig. 1. The observation unit optical system 30 is of a Cassegrain type as illustrated in Fig. 4. A primary mirror 31 and a secondary mirror 32 are arranged at the side opposed to the holding means 15 (the upper portion in the same figure). The pulsed light incident from above in the figure is reflected by the secondary mirror 32 and then reflected by the primary mirror 31 and travels toward the holding means 15. The focal point resides at the location of the holding means 15, and the biological tissue handled as the sample is arranged at this location. The transmitted pulsed light that has transmitted through the biological tissue further transmits through the holding means 15 and travels downward, and is guided to the detection-side optical path. It should be noted that, when the reflected pulsed light that has been reflected by the biological tissue is to be used, the reflected pulsed light is reflected by the primary mirror 31 and the secondary mirror 32, then travels toward the incidence side (the upper portion in the figure) and guided to the detection-side optical path.

Fig. 5 illustrates a specific configuration of the holding means 15. As illustrated in this figure, the holding means 15 includes a supporting substrate 40, a metamaterial 41 arranged on the supporting substrate 40, and an insulating film 42 arranged on the metamaterial 41 such as silicon dioxide ($SiO_2$), etc. Also, a piezoelectric element (vibration device) 43 such as a PZT element, etc. is provided on the side portion on the insulating film 42. In the same figure, the sample (biological tissue) S placed on the insulating film 42 is also illustrated. The sample S is cut into a thickness of several micrometers to several tens of micrometers, for example.

The metamaterial 41 is a substance in which minute units called "unit elements" are artificially arranged with equal intervals at distances sufficiently smaller than the wavelength of electromagnetic waves and the metamaterial 41 is configured to behave as a homogeneous medium for electromagnetic waves.

A not-shown power feeding cable and a not-shown control signal cable are connected to the piezoelectric element 43, and the piezoelectric element 43 is controlled by the control means 14. The piezoelectric element 43 is configured to be subjected to sweeping in accordance with a command from the control means 14, with a vibration frequency f [kHz] within a predetermined range, for example, from several kilohertz to several hundred hertz. The vibration frequency of the piezoelectric element 43 at the time of the measurement is captured in a storage area of the control means 14 along with the elapsed time. It should be noted that, while the piezoelectric elements 43 are provided on both sides of the insulating film 42 in Fig. 5, the number of the piezoelectric elements may be one or three or more as long as it is capable of imparting vibrations to the sample S, and the locations of installation thereof are not limited to the side portion.

As illustrated in Fig. 1, an optical delay means 16 for time origin adjustment and an optical delay means 17 for time-series signal measurement are arranged on a detection-side optical path extending from the beam splitter 2 to the detection element 4. The order of arrangement of the optical delay means 16 for time origin adjustment and the optical delay means 17 for time-series signal measurement may be inverted.

The optical delay means 16, 17 each include two corner cube mirrors 18. The corner cube mirror 18 is fixed to an automatic feeding stage driven along a single axis and configured to change in a stepwise manner (or successively) the length of the light path from the beam splitter 2 to the detection element 4 by virtue of the stage feeding. The movement of the stage can be measured by the He-Ne laser 19.

The optical delay means 16, 17 having the above-described configuration have the characteristic that the length of the light path can be changed two times as large as that in a case of one single corner cube mirror for scanning of the corner cube mirror. Accordingly, the effect achieved by these means is that it is made possible to specify settings for prompt time origin adjustment and the time-series signal measurement.

A driving device (trigger generation circuit) 20 that automatically carries out scanning is connected to the optical delay means 16 for time origin adjustment and the optical delay means 17 for time-series signal measurement, and the control means 14 that automatically controls the driving device 20 is also connected thereto.

An elliptical mirror (aspherical mirror) 21 and a plane mirror 22 are provided as optical elements on the incidence-side light path between the terahertz wave generation element 3 and the holding means 15. The elliptical mirror 21 is configured to concentrate the pulsed light from the terahertz wave generation element 3. The plane mirror 22 is arranged in the light path between the terahertz wave generation element 3 and the elliptical mirror 21 and has the redirection function for redirecting the pulsed light from the terahertz wave generation element 3. It should be noted that, while one elliptical mirror 21 and one plane mirror 22 are provided as in the case of the present embodiment, multiple elliptical mirrors 21 and multiple plane mirrors 22 can be used in combination.

An elliptical mirror (aspherical mirror) 23 and a plane mirror 24 are provided as optical elements on the detection-side optical path between the detection element 4 and the holding means 15. The elliptical mirror 23 is configured to concentrate the transmitted pulsed light from the sample. The plane mirror 24 is arranged on the light path between the elliptical mirror 23 and the detection element 4, and has the redirection function for redirecting the transmitted pulsed light from the elliptical mirror 23. It should be noted that, while one elliptical mirror 23 and one plane mirror 24 are provided as in the case of the present embodiment, multiple elliptical mirrors 23 and multiple plane mirrors 24 can be used in combination.

A current voltage conversion means (AVC) 25 is connected to the detection element 4. The AVC 25 is configured to convert the current signal that has been detected by the detection element 4 into a voltage signal.

A control means 14 is connected to the modulation means 13 and the current voltage conversion means 25 directly and via the lock-in amplifier 26. The control means 14 is capable of controlling the application of voltage to the antenna electrode films by the modulation means 13.

The control means 14 includes an analog-to-digital (A/D) converter 14a, an information processing unit 14b, and an input/output unit 14c. The information processing unit 14b is configured by, for example, a central processing unit (CPU), a random access memory (RAM), a read only memory (ROM), a computer readable storage medium, and the like. In addition, the series of processes for realizing the various functions are as an example stored in a storage medium or the like in the form of a program, and the various functions are realized by the CPU reading the program from the RAM or the like and carrying out information processing and arithmetic processing. It should be noted that various modes may be implemented for the program, such as a mode according to which the program is installed in advance in the ROM or another storage medium, a mode according to which the program is provided in a state where the program is stored in a computer-readable storage medium, and a mode according to which the program is distributed by wired or wireless communication means. The computer-readable storage medium refers to a magnetic disk, a magneto-optical disk, a CD-ROM disc, a DVD-ROM disc, a semiconductor memory, and the like.

The control means 14 is configured to control the device for observing the dynamic physical property of the biological tissue and has the functions for computing and displaying data acquired by measurements.

Next, the operation of the device for observing the dynamic physical property of the biological tissue according to the present embodiment will be described.

The pulsed laser light $L_1$ emitted from the pulsed laser light source 1 is split by the beam splitter 2 into the pulsed laser light (pump pulsed light) $L_2$ for use in excitation and the pulsed laser light (sampling pulsed light) $L_3$ for use in detection.

The excitation pulsed laser light $L_2$ is emitted via the lens 27 to the terahertz wave generation element 3. At this point, the excitation pulsed laser light $L_2$ is concentrated onto the photoconductive film provided in the gap 8 of the antenna electrode film. In a state where bias voltage is applied to the antenna electrode film, when the photoconductive film is irradiated with the excitation pulsed laser light $L_2$, the current due to the light excitation flows instantaneously, and a far-infrared electromagnetic pulse (pulsed light) is emitted.

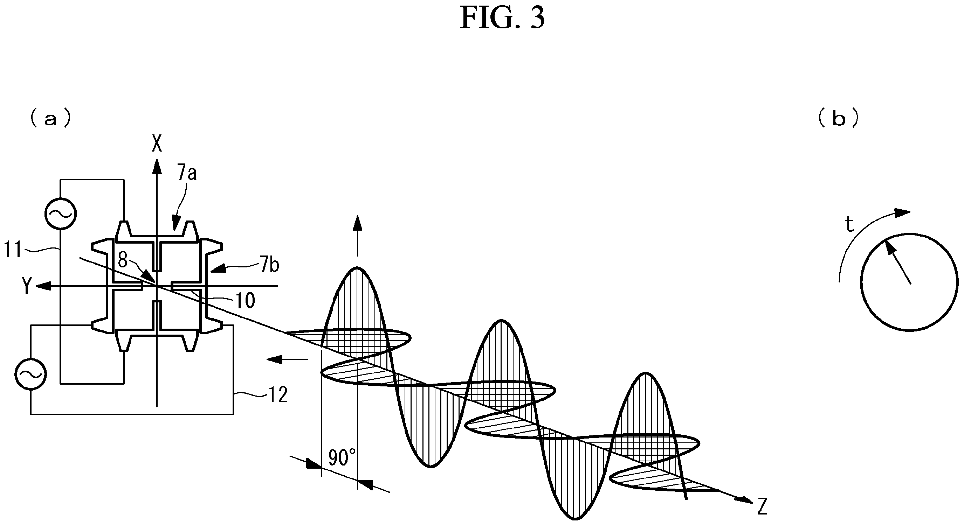

At this point, the modulation means 13, in accordance with a command issued from the information processing unit 14b, simultaneously applies bias voltages to the first antenna electrode film 7a and the second antenna electrode film 7b, where the bias voltages have the same amplitude and period but the different phases shifted relative to each other, and modulates the pulsed light into a circular polarized light (see (a) and (b) of Fig. 3).

When not only the phase but the amplitude of the sinusoidal voltage applied to the second antenna electrode film 7b are differentiated, the pulsed light in the form of an elliptically polarized light will be emitted. By such a use, vibrational circular dichroism measurement can be performed.

The pulsed light, after its optical path is redirected by the plane mirror 22, is guided to the elliptical mirror 21 and concentrated so that the sample S is irradiated therewith. At this point, vibrations are forcibly imparted to the sample S by the piezoelectric element 43. The control means 14 carries out the sweeping with a vibration frequency f [kHz] within a predetermined range. In the presence of the metamaterial 41, the sample S is irradiated with the pulsed light and accordingly an increased amount of optical information will be transmitted or reflected.

The transmitted pulsed light that has transmitted the sample S and carries the optical information of the sample (or a reflected pulsed light that has been reflected by the sample) is reflected by the elliptical mirror 23 and then redirected by the plane mirror 24, and further guided to the detection element 4. At this point, the reflected or transmitted pulsed light is concentrated onto the photoconductive film residing in the gap 8 of the antenna electrode films.

The detection pulsed laser light $L_3$ resulting from the splitting by the beam splitter 2 is given a delay time difference at predetermined time intervals by the optical delay means 16 for time origin adjustment and the optical delay means 17 for time-series signal measurement and guided to the detection element 4. At this point, the detection pulsed laser light $L_3$ is concentrated onto the photoconductive film residing in the gap 8 of the antenna electrode films, and superposed on the reflected or transmitted pulsed light of the sample.

The detection element 4 will exhibit conductivity for the moment during which the photoconductive film is irradiated with the detection pulsed laser light $L_3$. Hence, the electric field intensity and the phase lead of the reflected or transmitted pulsed light from the sample to which a trigger is applied and which has arrived at the moment at which the detection element 4 has become conductive are detected as the current signal.

The current signal that has been detected by the detection element 4 is converted into a voltage signal and amplified by the current voltage conversion means 25 and the voltage signal is sent to the lock-in amplifier 26. The pulsed light emitted from the terahertz wave generation element 3 is modulated by the modulation means 13, and the lock-in amplifier 26 uses the cyclic frequency of the modulated pulsed light as the reference signal, picks up only the voltages synchronized with the reference signal using a frequency filter, and the signal is detected with the influence of the background noise decreased.

The voltage signal that has been amplified by the lock-in amplifier 26 is converted into a digital signal by the A/D converter 14a. The information processing unit 14b performs Fourier transformation on the digital signal and calculates the amplitude of the electric field intensity and the spectroscopic spectrum of the phase of the reflected or transmitted pulsed light of the sample.

The control means 14 obtains the polarization spectrum by standardizing spectroscopic spectra that have been obtained or obtaining the difference of them using the spectrum in a case where the sample does not exist. The control means 14 obtains the vibrational circular dichroism spectrum (VCD spectrum) by obtaining the difference of the left and right polarized lights of the spectroscopic spectra that have been obtained. The control means 14 can obtain the dynamic physical property of the sample based on the polarization spectrum and the vibrational circular dichroism spectrum that have been obtained, and observe them. The dynamic physical property may include elastic modulus ($\eta$), dielectric constant ($\varepsilon$), relaxation time (T1), refractive index.

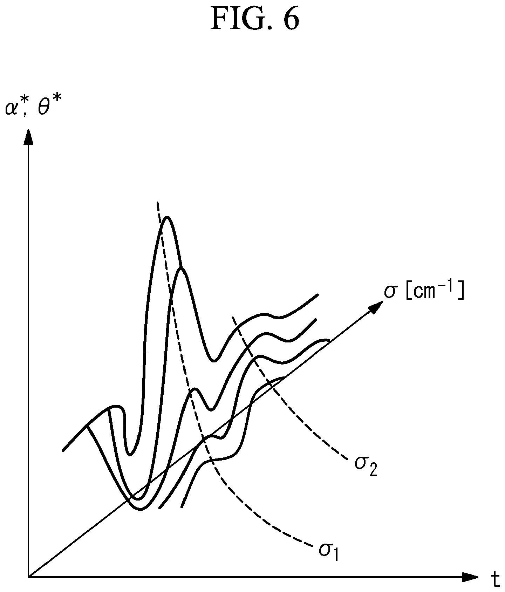

Fig. 6 illustrates a VCD spectrum of an optical rotatory dispersion $\alpha^*(\sigma)$ or a circular polarization dichroism ellipticity $\theta^*(\sigma)$ which are examples of optically active amount or optical physical property amount acquired by the above-described device for observing the dynamic physical property of the biological tissue. Here, σ represents the wave number. In this figure, the horizontal axis represents time t, the vertical axis represents the optical rotatory dispersion $\alpha^*(\sigma)$ or circular polarization dichroism ellipticity $\theta^*(\sigma)$, and the depth axis represents the wave number $\sigma[cm^{-1}]$. The graph illustrated in Fig. 6 can be computed by the control means 14 to be displayed.

As can be appreciated from the same figure, the waveform whose maximum and minimum are conspicuously distinct from each other at the beginning of the measurement is gradually attenuated with the passage of time. And the relaxation time T can be obtained from the attenuation of the wave numbers σ1 and σ2 representing the maximum value. For example, the relaxation time T of the optical rotatory dispersion $\alpha^*(\sigma)$ can be obtained from the following expression:

$$\alpha^*(\sigma) = \exp(t/T)$$

where t is the elapsed time.

Next, data processing using the two-dimensional correlation spectroscopy will be described. For the general understanding of the two-dimensional correlation spectroscopy, "Experimental Infrared Spectroscopy Fundamentals and Practical Methods" (written and edited by Mitsuo Tasumi, Spectroscopical Society of Japan; S.T. Japan INC. April 2012), "Part II Various Measurement Methods 21. Two-Dimensional Correlation Spectroscopy" (pages 158 to 165) may be referred to. The data illustrated in Fig. 6 is obtained for each frequency used when the sample S is vibrated with the vibration frequency f[kHz] by the piezoelectric element 43. By virtue of this, the optically active amount can be measured in accordance with the vibration frequency f.

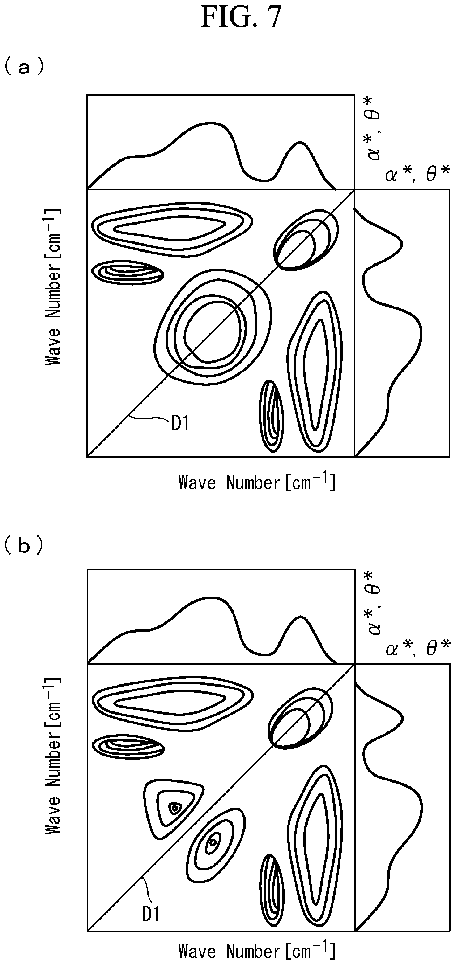

In addition, as illustrated in Fig. 7, the synchronous correlation spectrum (Fig. 7(a)) and the asynchronous correlation spectrum (Fig. 7(b)) are obtained. The individual figures in Fig. 7 describe the spectrum of the optical rotatory dispersion $\alpha^*(\sigma)$ or the circular polarization dichroism ellipticity $\theta^*(\sigma)$ on the right side and the upper side of the respective graphs. The respective graphs illustrated in Fig. 7 can be computed by the control means 14 to be displayed.

Referring to Fig. 7(a) illustrating contour lines of the synchronous correlation spectrum, the spectrum is symmetrical to the diagonal line D1. In contrast, referring to Fig. 7(b) illustrating the contour lines of the asynchronous correlation spectrum, it can be seen that the symmetry to the diagonal line D1 is lost. In view of this, it is possible to determine that a certain structural change has occurred with a certain wave number in the particular vibration frequency f. In this manner, it is made possible to provide the basic data for elucidating the structure related, for example, to water molecule of the biological tissue.

As has been discussed in the foregoing, by externally and forcibly imparting vibrations to give the elastic wave, the differences of dynamic perturbative variation among multiple biological tissues can be observed as an amount of change appearing in the optically active amount or optical physical property amount.

According to the present embodiment, the following effects can be obtained.

When the sample S which is a biological tissue is irradiated with the pulsed light, the biological tissue is vibrated by the piezoelectric element 43. By forcibly imparting vibrations to the biological tissue to cause the biological tissue to vibrate, it is made possible to perform spectroscopic measurement in accordance with the vibration frequency for imparting the vibrations, and obtain an increased amount of information on the biological tissue at the molecule level.

Since the vibration frequency by the piezoelectric element 43 is swept within a predetermined range, it is made possible to identify the structure of a biological molecule in accordance with various frequencies.

When the relaxation time T of the biological tissue is to be obtained as the dynamic physical property, the vibration frequency dependence of the relaxation time T can be revealed, which contributes to the elucidation of the structure of the biological tissue.

[Reference Signs List]

| | |
|---|---|
| 1 | pulsed laser light source |
| 2 | beam splitter (splitting means) |
| 3 | terahertz wave generation element (radiation means) |
| 4 | detection element (detection means) |
| 5, 6 | super-hemispherical silicon lens |
| 7a | first antenna electrode film |
| 7b | second antenna electrode film |
| 8 | gap |
| 9 | conductive transmission channel |
| 10 | discharge electrode |
| 11 | first lead |
| 12 | second lead |
| 13 | modulation means |
| 14 | control means |
| 14a | A/D converter |
| 14b | information processing unit |
| 14c | input/output unit |
| 15 | holding means |
| 16 | optical delay means (for time origin adjustment) |
| 17 | optical delay means (for time-series signal measurement) |
| 18 | corner cube mirror |
| 19 | He-Ne laser |
| 20 | driving device (trigger generation circuit) |
| 21, 23 | elliptical mirror (aspherical mirror) |
| 22, 24 | plane mirror |
| 25 | current voltage conversion means |

26      lock-in amplifier
27      lens
30      observation unit optical system
41      metamaterial
43      piezoelectric element (vibration device)
S       sample (biological tissue)

**Claims**

1. A method for observing a dynamic physical property of a biological tissue by irradiating the biological tissue with a pulsed light having a wavelength of a far-infrared wavelength region to observe the dynamic physical property of the biological tissue using vibrational optical activity spectroscopy, the method comprising vibrating the biological tissue by vibrating a holding means (15) using a piezoelectric element (43), the holding means configured to hold the biological tissue when the biological tissue is irradiated with the pulsed light.

2. The method for observing the dynamic physical property of the biological tissue according to claim 1, the method comprising:

   using a radiation means (3) having

      a photoconductive film receiving a pulse excitation light and generating a photocarrier and
      a pair of first antenna electrode films (7a) formed on the photoconductive film and facing each other via a gap (8), a pair or multiple pairs of second antenna electrode films (7b) formed on the photoconductive film and arranged so as to face each other via the gap (8) and arranged so as to have an angle relative to the first antenna electrode films (7a), and

   being configured to emit the pulsed light having the wavelength of the far-infrared wavelength region by pulsed laser light irradiation;
   irradiating the biological tissue with the pulsed light from the radiation means (3) while vibrating the biological tissue; and
   observing the dynamic physical property of the biological tissue based on a vibrational circular dichroism spectrum and/or polarization spectroscopy spectrum obtained from a time-series signal of a reflected pulsed light reflected by the biological tissue or a transmitted pulsed light transmitted through the biological tissue.

3. A device for observing a dynamic physical property of a biological tissue, the device comprising

   a radiation means (3) including

      a photoconductive film receiving an excitation pulsed laser light and generating a photocarrier and
      a pair of first antenna electrode films (7b) formed on the photoconductive film and facing each other via a gap (8), and

   configured to emit a pulsed light having a wavelength of a far-infrared wavelength region by irradiation of the excitation pulsed laser light;
   a holding means (15) configured to hold the biological tissue;
   a vibration device configured to vibrate the biological tissue by vibrating the holding means (15) using a piezoelectric element when the biological tissue is irradiated with the pulsed light;
   a detection means (4) including a pair or multiple pairs of second antenna electrode films (7b) arranged so as to face each other via the gap (8) and arranged so as to have an angle relative to the first antenna electrode films (7a), the detection means (4) being configured to obtain a time-series signal of a reflected pulsed light reflected by the biological tissue or a transmitted pulsed light transmitted through the biological tissue; and
   a control means (14) configured to observe the dynamic physical property of the biological tissue using vibrational optical activity spectroscopy, based on a vibrational circular dichroism spectrum and/or polarization spectroscopy spectrum obtained from a signal obtained from the detection means (4).

**Patentansprüche**

1. Verfahren zur Beobachtung einer dynamisch-physikalischen Eigenschaft von biologischem Gewebe durch Bestrahlen des biologischen Gewebes mit einem gepulsten Licht mit einer Wellenlänge eines Ferninfrarot-Wellenlängenbereichs, um die dynamisch-physikalische Eigenschaft des biologischen Gewebes mittels Vibrationsspektroskopie mit optischer Aktivität zu beobachten, wobei das Verfahren umfasst, dass das biologische Gewebe in Vibration versetzt wird, indem eine Halteeinrichtung (15) mit Hilfe eines piezoelektrischen Elements (43) in Vibration versetzt wird, wobei die Halteeinrichtung ausgebildet ist zum Halten des biologischen Gewebes, wenn das biologische Gewebe mit dem gepulsten Licht bestrahlt wird.

2. Verfahren zur Beobachtung einer dynamisch-physikalischen Eigenschaft von biologischem Gewebe nach Anspruch 1, wobei das Verfahren umfasst:

Verwenden einer Bestrahlungseinrichtung (3) mit einem fotoleitenden Film, der ein Pulsanregungslicht empfängt und einen Fototräger erzeugt, und

einem Paar von ersten Antennenelektrodenfilmen (7a), die auf dem fotoleitenden Film gebildet und einander über einen Spalt (8) zugewandt sind,

einem oder mehreren Paaren von zweiten Antennenelektrodenfilmen (7b), die auf dem fotoleitenden Film gebildet und derart angeordnet sind, dass sie einander über den Spalt (8) zugewandt sind, und derart, dass sie relativ zu den ersten Antennenelektrodenfilmen (7a) einen Winkel aufweisen,

und eingerichtet zum Aussenden des gepulsten Lichts mit der Wellenlänge des Ferninfrarot-Wellenlängenbereichs durch gepulste Laserlichtbestrahlung;

Bestrahlen des biologischen Gewebes mit dem gepulsten Licht aus der Bestrahlungseinrichtung (3) unter Vibrieren des biologischen Gewebes; und

Beobachten der dynamisch-physikalischen Eigenschaft des biologischen Gewebes basierend auf einem Vibrations-Zirkulardichroismusspektrum und/oder einem Polarisationsspektroskopiespektrum, das aus einem Zeitseriensignal eines von dem biologischen Gewebe reflektierten gepulsten Lichts oder eines durch das biologische Gewebe transmittierten gepulsten Lichts gewonnen wird.

**3.** Vorrichtung zur Beobachtung einer dynamisch-physikalischen Eigenschaft von biologischem Gewebe, wobei die Vorrichtung umfasst

eine Bestrahlungseinrichtung (3) mit einem fotoleitenden Film, der ein gepulstes Anregungslaserlicht empfängt und einen Fototräger erzeugt, und

einem Paar von ersten Antennenelektrodenfilmen (7b), die auf dem fotoleitenden Film gebildet und einander über einen Spalt (8) zugewandt sind, und

ausgebildet zum Aussenden eines gepulsten Lichts mit einer Wellenlänge des Ferninfrarot-Wellenlängenbereichs durch Bestrahlung des gepulsten Anregungslaserlichts; eine Halteeinrichtung (15), die eingerichtet ist zum Halten des biologischen Gewebes;

eine Vibrationseinrichtung, die dazu eingerichtet ist, das biologische Gewebe in Vibration zu versetzen, indem die Halteeinrichtung (15) mit Hilfe eines piezoelektrischen Elements in Vibration versetzt wird, wenn das biologische Gewebe mit dem gepulsten Licht bestrahlt wird;

eine Detektionseinrichtung (4) mit einem oder mehreren Paaren von zweiten Antennenelektrodenfilmen (7b), die derart angeordnet sind, dass sie einander über den Spalt (8) gegenüberliegen, und derart, dass sie relativ zu den ersten Antennenelektrodenfilmen (7a) einen Winkel aufweisen, wobei die Detektionseinrichtung (4) dazu eingerichtet ist, ein Zeitseriensignal eines reflektierten gepulsten Lichts, das von dem biologischen Gewebe reflektiert wird, oder eines transmittierten gepulsten Lichts, das durch das biologische Gewebe transmittiert wird, zu gewinnen; und

eine Steuereinrichtung (14), die dazu eingerichtet ist, die dynamisch-physikalische Eigenschaft des biologischen Gewebes mittels Vibrationsspektroskopie mit optischer Aktivität zu beobachten, basierend auf einem Vibrations-Zirkulardichroismusspektrum und/oder einem Polarisationsspektroskopiespektrum, das aus einem Signal erhalten wird, das von der Detektionseinrichtung (4) erhalten wird.

## Revendications

**1.** Procédé d'observation de propriété physique dynamique de tissu biologique en irradiant le tissu biologique avec une lumière pulsée ayant une longueur d'onde d'une gamme de longueurs d'onde de l'infrarouge lointain afin d'observer la propriété physique dynamique du tissu biologique au moyen d'une spectroscopie de vibration avec une activité optique, le procédé comprenant la mise en vibration du tissu biologique en faisant vibrer un dispositif de maintien (15) au moyen d'un élément piézoélectrique (43), le dispositif de maintien étant adapté pour maintenir le tissu biologique lorsque le tissu biologique est irradié par la lumière pulsée.

**2.** Procédé d'observation de propriété physique dynamique de tissu biologique selon la revendication 1, le procédé comprenant

utiliser un dispositif d'irradiation (3) comprenant un film photoconducteur qui reçoit une lumière d'excitation pulsée et produit un photoporteur, et une paire de premiers films d'électrodes d'antenne (7a) formés sur le film photoconducteur et se faisant face l'un l'autre via un espace (8), une ou plusieurs paires de deuxièmes films d'électrodes d'antenne (7b) formées sur le film photoconducteur et disposées de manière à se faire face à travers l'espace (8) et de manière à former un angle par rapport aux premiers films d'électrodes d'antenne (7a), et agencé pour émettre la lumière pulsée ayant la longueur d'onde de la gamme de longueurs d'onde de l'infrarouge lointain par irradiation de lumière laser pulsée;

irradiation du tissu biologique avec la lumière pulsée provenant du dispositif d'irradiation (3), tout en faisant vibrer le tissu biologique; et

observer la propriété physique dynamique du tissu biologique sur la base d'un spectre de dichroïsme circulaire de vibration et/ou d'un spectre de spectroscopie de polarisation obtenu à partir d'un signal de série temporelle d'une lumière pulsée réfléchie par le tissu biologique ou d'une lumière pulsée transmise à travers le tissu biologique.

3. Dispositif d'observation de propriété physique dynamique de tissu biologique, le dispositif comprenant un dispositif d'irradiation (3) comprenant un film photoconducteur qui reçoit une lumière laser d'excitation pulsée et produit un photoporteur, et

une paire de premiers films d'électrodes d'antenne (7b) formés sur le film photoconducteur et se faisant face par l'intermédiaire d'un espace (8), et

conçu pour émettre une lumière pulsée ayant une longueur d'onde de la gamme de longueurs d'onde de l'infrarouge lointain en irradiant la lumière laser d'excitation pulsée;

un dispositif de maintien (15) adapté pour maintenir le tissu biologique;

un moyen de vibration adapté pour faire vibrer le tissu biologique en faisant vibrer le moyen de support (15) au moyen d'un élément piézoélectrique lorsque le tissu biologique est irradié par la lumière pulsée;

un moyen de détection (4) comprenant une ou plusieurs paires de seconds films d'électrodes d'antenne (7b) disposés de manière à se faire face à travers l'espace (8) et de manière à former un angle par rapport aux premiers films d'électrodes d'antenne (7a), le moyen de détection (4) étant adapté pour obtenir un signal de série temporelle d'une lumière pulsée réfléchie par le tissu biologique ou d'une lumière pulsée transmise par le tissu biologique; et

un dispositif de commande (14) adapté pour observer la propriété physique dynamique du tissu biologique au moyen d'une spectroscopie de vibration avec activité optique, sur la base d'un spectre de dichroïsme circulaire de vibration et/ou d'un spectre de spectroscopie de polarisation obtenu à partir d'un signal obtenu par le dispositif de détection (4).

FIG. 1

EP 3 441 749 B1

# FIG. 2

# FIG. 3

（a）

（b）

EP 3 441 749 B1

# FIG. 4

# FIG. 5

# FIG. 6

# FIG. 7

(a)

(b)

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001141567 A
- US 2015008326 A1
- US 2014224021 A1
- EP 1801939 A1

**Non-patent literature cited in the description**

- **A. Y. ELEZZABI et al.** Optical activity in an artificial chiral media: a terahertz time-domain investigation of Karl F Lindman's 1920 pioneering experiment. *Optics Express,* 13 April 2009, vol. 17 (8), 6600-6612
- Development and versatile applications of terahertz time-domain spectroscopy. **HANGYO MASANORI.** 2014 39TH INTERNATIONAL CONFERENCE ON INFRARED, MILLIMETER, AND TERAHERTZ WAVES (IRMMW-THZ). IEEE, 14 September 2014, 1-4
- Part II Various Measurement Methods 21. Two-Dimensional Correlation Spectroscopy. Experimental Infrared Spectroscopy Fundamentals and Practical Methods. Spectroscopical Society of Japan; S.T. Japan INC, April 2012, 158-165